# EUROPEAN PATENT APPLICATION

(11) **EP 4 265 713 A1**
(43) Date of publication of application: **25.10.2023**
(21) Application number: 22169245.2
(22) Date of filing: 21.04.2022
(51) Int. Cl.: C12N 1/32, C12N 15/52, C12N 15/74, C12P 7/56, C12N 9/04

(54) **GENETICALLY MODIFIED METHYLOTROPHIC BACTERIA PRODUCING LACTATE**

(71) Applicant: Acies Bio d.o.o., 1000 Ljubljana (SI)
(72) Inventor: KRUIS, Aleksander Johannes, Bled (SI); VIRANT, David, Dob (SI); KRAJNC, Nika Lendero, alec (SI); FUJS, Stefan, Ljubljana (SI)
(74) Representative: Zacco GmbH

(57) **Abstract**

The present invention generally relates to the biotechnology engineering, and specifically to genetically modified methylotrophic bacteria which produce lactate. More specifically, the present invention provides a methylotrophic bacterium modified to have an increased expression of a polypeptide having lactate dehydrogenase activity. The present invention further provides a method for producing lactate using a genetically modified bacterium of the present invention.

## Description

### Technical field of the invention

The present invention generally relates to the biotechnology engineering, and specifically to genetically modified methylotrophic bacteria which produce lactate. More specifically, the present invention provides a methylotrophic bacterium modified to have an increased expression of a polypeptide having lactate dehydrogenase activity. The present invention further provides a method for producing lactate using a genetically modified bacterium of the present invention.

### Background of the invention

Lactic acid is a widely used building block for biodegradable polymer production. It is produced commercially by fermentation of carbohydrates by various bacteria, such as Bacillus coagulans. The carbohydrates, in particular C6 sugars, such as glucose are converted to lactate through an anaerobic or severely-oxygen limited process. The glucose is first imported into the host cell and metabolised to pyruvate, which is subsequently converted to lactate by the enzyme lactate dehydrogenase.

During fermentation, high lactate concentrations are produced, which is toxic to the production organism. To circumvent this problem, calcium hydroxide or calcium carbonate is used to regulate pH during the fermentation, which results in the formation of Ca-lactate salts. These are insoluble and precipitate during the fermentation, reducing the toxicity of the lactate. At the end of the process, the Ca-lactate is acidified with H₂SO₄ to convert it to lactic acid, which is then further purified to the final product, producing gypsum (CaSO₄) as a by-product.

Two enantiomers of lactate can be produced by microorganisms, D-lactate, and L-lactate. The stereospecificity of the lactate is determined by the lactate dehydrogenase that converts pyruvate to lactate and is generally organism specific. By replacing an L-lactate producing LDH with a D-lactate producing LDH through genetic methods, the final lactate form produced by the host can be controlled. L-lactic acid is currently the main commercially produced lactate form. Both lactic acid enantiomers can be polymerized into polylactic aci (PLA) to form L-PLA and D-PLA, which can be mixed subsequently to form DL-PLA.

Bio-based products, such as lactic acid, can be produced from various substrates, such as carbohydrates and methanol. Carbohydrates have been the dominant substrate used for microbial chemical production due to their widespread availability. While carbohydrates may be a sustainable substrate in terms of atmospheric CO₂ release, they compete with food and feed chains. Because of the growing global population, carbohydrate-derived substrates will become less and less sustainable in the long term. On the other hand, bio-based methanol production from wood biomass or CO₂-sequestration does not interfere with food and feed production.

Bacteria that can utilize methanol for growth and product formation (methylotrophs) have been identified. Examples include *Bacillus methanolicus, Methylobacterium extorquens, Methylobacillus glycogenes* and *Methylobacillus flagellatus.* Methylotrophic bacteria can be divided into two broad groups based on the underlying metabolism that they use to assimilate methanol. In both groups, methanol is first converted to formaldehyde using a methanol dehydrogenase. The first group, which includes M. *extorquens,* assimilate formaldehyde by reacting it with glycine, forming serine in the pathway known as the serine cycle.

The second group of methylotrophic bacteria use the Ribulose-monophosphate (RuMP) cycle to react formaldehyde with RuMP to form C6-compounds that are then metabolized further. *B*. *methanolicus* and Methylobacilli belong to the RuMP cycle group of methylotrophs. The RuMP pathway has two sub-variants, the Eda pathway and the Fba pathway. The FBA variant depends on the fructose bisphospate aldolase (Fba) enzyme to cleave the C6 intermediates of the RuMP cycle. This generates two GAP moieties, one of which is used for RuMP regeneration, and the other exists the cycle and enters lower glycolysis. The Eda dependent RuMP cycle uses the 2-keto-3-deoxy gluconate-6P aldolase (2,3-KDPG aldolase, or Eda for Entner-Doudorff pathway aldolase) to extract C3 compounds. The Eda enzyme splits the C6 compound 2-keto, 3-deoxy gluconate-6P (2,3-KDPG) into glyceraldehyde-3P (GAP) and pyruvate. GAP is used to replenish the RuMP cycle for the next cycle of methanol assimilation, while pyruvate exits the cycle.

Both groups of methylotrophic bacteria have been used to produce various biochemicals from methanol. However, lactate production with methylotrophs has not yet been reported. Many glucose-utilizing organisms such as *Escherichia coli* or lactic acid bacteria produce lactate under anaerobic conditions. In the absence of oxygen, lactate acts as the final acceptor of electrons released during glucose oxidation. This allows the organisms to produce ATP and prevents a complete halt in their metabolism. Oxygen limitation accompanied by overabundance of a carbon source can also lead to lactate production, in which case it is considered an "overflow" metabolite.

This is not possible in methylotrophs since they are obligate aerobes and require oxygen for methanol assimilation. Furthermore, methylotrophs have the option of generating ATP from methanol before it is assimilated through the RuMP or serine cycles via formaldehyde dissimilation pathways or during the RuMP cycle via the dissimilatory RuMP pathway. Both pathways generate NADH, which can enter the respiratory chain to produce ATP. Any carbon that is not needed for biomass formation is therefore converted into energy and CO2 before it even enters the central carbon metabolism. It is therefore highly unexpected that methylotrophs would efficiently produce lactate as glucose-utilizing organisms do.

### Summary of the invention

The object of the present invention is to provide means allowing efficient production of lactate at higher nominal yield. This is achieved by the present inventors who have engineered genetically modified methylotrophic bacteria which produce lactate.

More specifically, the present inventors have engineered methylotrophic bacterial strains, which have an increased expression of a polypeptide having lactate dehydrogenase activity. As shown in the Examples, such engineered bacterial strains surprisingly show unusually high titers of lactate in the supernatant.

The present invention this provides in a first aspect a methylotrophic bacterium which has been to have an increased protein expression of a polypeptide having lactate dehydrogenase activity compared to an otherwise identical bacterium that does not carry said modification.

The present invention further provides in a second aspect a method for producing lactate, comprising cultivating a bacterium according to the present invention under suitable culture conditions in a suitable culture medium.

The present invention may be further summarized by the following items:
1. A genetically engineered methylotrophic bacterium which has been modified to have an increased protein expression of a polypeptide having lactate dehydrogenase activity compared to an otherwise identical bacterium that does not carry said modification.
2. The bacterium according to item 1, wherein the increase in protein expression of the polypeptide having lactate dehydrogenase activity is achieved by increasing the number of copies of a nucleotide sequence encoding said polypeptide having lactate dehydrogenase activity.
3. The bacterium according to item 2, wherein the increase in the number of copies of the nucleotide sequence encoding said polypeptide is achieved by introducing into the bacterium at least one exogenous nucleic acid molecules comprising at least one nucleotide sequence encoding said polypeptide having lactate dehydrogenase activity.
4. The bacterium according to any one of items 1 to 3, wherein the bacterium comprises at least one exogenous nucleic acid molecule (such as a vector) comprising at least one nucleotide sequence encoding the polypeptide having lactate dehydrogenase activity.
5. The bacterium according to item 3 or 4, wherein the exogenous nucleic acid molecule comprises at least one transcriptional unit comprising, from 5' to 3', a promoter that is functional in the bacterium to cause the production of an mRNA molecule and that is operably linked to a nucleotide sequence encoding said polypeptide having lactate dehydrogenase activity, and a transcriptional terminator sequence.
6. The bacterium according to any one of items 3 to 5, wherein the exogenous nucleic acid molecule is a vector, such as a plasmid.
7. The bacterium according to any one of items 3 to 5, wherein the exogenous nucleic acid molecule is stably integrated into the genome of the bacterium.
8. The bacterium according to any one of items 1 to 7, wherein the increase in protein expression of the polypeptide having lactate dehydrogenase activity is achieved by modifying the ribosome binding site of an endogenous gene encoding the polypeptide having lactate dehydrogenase activity.
9. The bacterium according to any one of items 1 to 8, wherein the increase in protein expression of the polypeptide having lactate dehydrogenase activity is achieved by increasing the strength of the promoter operably linked to an endogenous gene encoding the polypeptide having lactate dehydrogenase activity.
10. The bacterium according to any one of items 1 to 9, wherein the polypeptide having lactate dehydrogenase activity is selected from the group consisting of: i) a polypeptide comprising an amino acid sequence of any one of SEQ ID NOs: 1 to 130 and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity with the amino acid sequence of any one of SEQ ID NOs: 1 to 130.
11. The bacterium according to any one of items 1 to 10, wherein the polypeptide having lactate dehydrogenase activity is selected from the group consisting of: i) a polypeptide comprising an amino acid sequence of any one of SEQ ID NOs: 1 to 48; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity with the amino acid sequence of any one of SEQ ID NOs: 1 to 48.
12. The bacterium according to any one of items 1 to 11, wherein the polypeptide having lactate dehydrogenase activity is selected from the group consisting of: i) a polypeptide comprising the amino acid sequence of SEQ ID NO: 1; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity with the amino acid sequence of SEQ ID NO: 1.
13. The bacterium according to any one of items 1 to 12, wherein the polypeptide having lactate dehydrogenase activity is a heterologous polypeptide having lactate dehydrogenase activity.
14. The bacterium according to any one of items 1 to 13, which has been further modified to have a decreased expression and/or activity of an endogenous polypeptide having polyphosphate kinase activity compared to an otherwise identical bacterium that does not carry said modification.
15. The bacterium according to any one of items 1 to 14, which has been modified to have a decreased expression of an endogenous polypeptide having polyphosphate kinase activity compared to an otherwise identical bacterium that does not carry said modification.
16. The bacterium according to item 14 or 15, wherein the expression level of the endogenous polypeptide having polyphosphate kinase activity is decreased by at least 50%, such as by at least 60%, at least 65%, at least 70%, at least 75%, at least 80%, at least 85%, at least 90%, at least 95% or at least 100% compared to the otherwise identical bacterium.
17. The bacterium according to any one of item 14 to 16, wherein the endogenous gene encoding said polypeptide having polyphosphate kinase activity has been inactivated.
18. The bacterium according to item 17, wherein the endogenous gene encoding said polypeptide having polyphosphate kinase activity has been inactivated by deletion of part of or the entire gene sequence.
19. The bacterium according to item 17 or 18, wherein the endogenous gene encoding said polypeptide having polyphosphate kinase activity has been inactivated by introducing or expressing in the bacterium a rare-cutting endonuclease able to selectively inactivating by DNA cleavage the endogenous gene encoding said polypeptide.
20. The bacterium according to item 19, wherein said rare-cutting endonuclease is a transcription activator-like effector (TALE) nuclease, meganuclease, zinc-finger nuclease (ZFN), or RNA-guided endonuclease.
21. The bacterium according to item 20, wherein the RNA-guided endonuclease is a catalytically inactive Cas9 protein.
22. The bacterium according to item 21, which comprises (e.g., expresses) a single guide RNA (sgRNA) specifically hybridizing (e.g. binding) under cellular conditions with the genomic DNA encoding said polypeptide.
23. The bacterium according to any one of items 14 to 16, wherein the expression of said endogenous polypeptide having polyphosphate kinase activity is decreased (e.g., inhibited) by transcriptional and/or translational repression of the endogenous gene encoding said polypeptide.
24. The bacterium according to any one of items 14 to 16, wherein the expression of said endogenous polypeptide having polyphosphate kinase activity is decreased (e.g. inhibited) by introducing or expressing in the bacterium an inhibitory nucleic acid molecule that specifically hybridizes (e.g. binds) under cellular conditions with cellular mRNA and/or genomic DNA encoding said polypeptide.
25. The bacterium according to item 24, wherein the inhibitory nucleic acid molecule is an antisense oligonucleotide, ribozyme or interfering RNA (RNAi) molecule.
26. The bacterium according to item 25, wherein the interfering RNA molecule is a micro RNA (miRNA), small interfering RNA (siRNA) or short hairpin RNA (shRNA).
27. The bacterium according to any one of items 1 to 14, which has been modified to have a decreased activity of an endogenous polypeptide having polyphosphate kinase activity compared to an otherwise identical microorganism that does not carry said modification.
28. The bacterium according to item 27, wherein the activity of said polypeptide is decreased by at least one active-site mutation resulting in the reduction or loss of activity.
29. The bacterium according to item 28, wherein the at least one active-site mutation is a non-conservative amino acid substitution.
30. The bacterium according to any one of items 1 to 29, which has been further modified to have a decreased expression and/or activity of an endogenous polypeptide having Acyl-homoserine-lactone (AHL) synthase activity compared to an otherwise identical bacterium that does not carry said modification.
31. The bacterium according to any one of items 1 to 30, which has been modified to have a decreased expression of an endogenous polypeptide having Acyl-homoserine-lactone (AHL) synthase activity compared to an otherwise identical bacterium that does not carry said modification.
32. The bacterium according to item 30 or 31, wherein the expression level of the endogenous polypeptide having Acyl-homoserine-lactone (AHL) synthase activity is decreased by at least 50%, such as by at least 60%, at least 65%, at least 70%, at least 75%, at least 80%, at least 85%, at least 90%, at least 95% or at least 100% compared to the otherwise identical bacterium.
33. The bacterium according to any one of item 30 to 32, wherein the endogenous gene encoding said polypeptide having Acyl-homoserine-lactone (AHL) synthase activity has been inactivated.
34. The bacterium according to item 33, wherein the endogenous gene encoding said polypeptide having Acyl-homoserine-lactone (AHL) synthase activity has been inactivated by deletion of part of or the entire gene sequence.
35. The bacterium according to item 33 or 34, wherein the endogenous gene encoding said polypeptide having Acyl-homoserine-lactone (AHL) synthase activity has been inactivated by introducing or expressing in the bacterium a rare-cutting endonuclease able to selectively inactivating by DNA cleavage the endogenous gene encoding said polypeptide.
36. The bacterium according to item 35, wherein said rare-cutting endonuclease is a transcription activator-like effector (TALE) nuclease, meganuclease, zinc-finger nuclease (ZFN), or RNA-guided endonuclease.
37. The bacterium according to item 36, wherein the RNA-guided endonuclease is a catalytically inactive Cas9 protein.
38. The bacterium according to item 37, which comprises (e.g., expresses) a single guide RNA (sgRNA) specifically hybridizing (e.g. binding) under cellular conditions with the genomic DNA encoding said polypeptide.
39. The bacterium according to item 30 or 31, wherein the expression of said endogenous polypeptide having Acyl-homoserine-lactone (AHL) synthase activity is decreased (e.g., inhibited) by transcriptional and/or translational repression of the endogenous gene encoding said polypeptide.
40. The bacterium according to item 30 or 31, wherein the expression of said endogenous polypeptide having Acyl-homoserine-lactone (AHL) synthase activity is decreased (e.g. inhibited) by introducing or expressing in the bacterium an inhibitory nucleic acid molecule that specifically hybridizes (e.g. binds) under cellular conditions with cellular mRNA and/or genomic DNA encoding said polypeptide.
41. The bacterium according to item 40, wherein the inhibitory nucleic acid molecule is an antisense oligonucleotide, ribozyme or interfering RNA (RNAi) molecule.
42. The bacterium according to item 41, wherein the interfering RNA molecule is a micro RNA (miRNA), small interfering RNA (siRNA) or short hairpin RNA (shRNA).
43. The bacterium according to item 30, which has been modified to have a decreased activity of an endogenous polypeptide having Acyl-homoserine-lactone (AHL) synthase activity compared to an otherwise identical microorganism that does not carry said modification.
44. The bacterium according to item 43, wherein the activity of said polypeptide is decreased by at least one active-site mutation resulting in the reduction or loss of activity.
45. The bacterium according to item 44, wherein the at least one active-site mutation is a non-conservative amino acid substitution.
46. The bacterium according to any one of items 1 to 45, which expresses a RuMP cycle.
47. The bacterium according to any one of items 1 to 46, which expresses an Eda (2-keto-3-deoxy-phosphogluconate aldolase)-dependent RuMP cycle.
48. The bacterium according to any one of items 1 to 47, which expresses a polypeptide having phosphoglucoisomerase activity and a polypeptide having 2-keto-3-deoxy-phosphogluconate aldolase activity.
49. The bacterium according to any one of items 1 to 48, wherein said bacterium belongs to the family *Methylophilaceae* or *Methylobacteriaceae.*
50. The bacterium according to any one of items 1 to 49, wherein said bacterium belongs to the genus *Methylobacillus, Methylobacterium or Methylorubrum, preferably Methylobacillus* or *Methylobacterium.*
51. The bacterium according to any one of items 1 to 50, which is selected from *Methylobacillus flagellatus*, *Methylobacillus glycogenes, Methylobacillus pratensis, Methylobacillus rhizosphaerae, Methylobacillus gramineus, Methylobacillus arboreus, Methylobacillus caricics, Methylobacillus methilovorans, Methylobacillus sp, Methylobacterium extorquens, Methylobacterium organophilum and Methylorubrum extorquens.*
52. The bacterium according to any one of items 1 to 50, wherein said bacterium is of the genus *Methylobacillus.*
53. The bacterium according to any one of items 1 to 50, which is selected from *Methylobacillus flagellatus*, *Methylobacillus glycogenes, Methylobacillus pratensis, Methylobacillus rhizosphaerae, Methylobacillus gramineus, Methylobacillus arboreus, Methylobacillus caricics, Methylobacillus methilovorans and Methylobacillus sp.*
54. The bacterium according to any one of items 1 to 50, wherein said bacterium is *Methylobacillus flagellatus.*
55. The bacterium according to any one of items 1 to 50, wherein said bacterium is *Methylobacillus glycogenes.*
56. Method for producing lactate comprising cultivating a bacterium according to any one of items 1 to 55 under suitable culture conditions.
57. The method according to item 56 comprising cultivating said bacterium under suitable culture conditions in a culture medium comprising a reduced one-carbon compound, such as methanol, or a multi-carbon compound that contains no carbon-carbon bonds, such as dimethyl ether and dimethylamine.
58. The method according to item 57, wherein the culture medium comprises methanol.
59. The method according to any one of items 56 to 58, wherein the cultivation is performed in a bioreactor.

### Brief description of the figures

**Figure 1****:** D-Lactate production from methanol by the strain OCB 354 that overexpressed the Mfla_0399 gene (SEQ ID NO: 137, encoding SEQ ID NO: 1).
**Figure 2****:** L-Lactate production from methanol by the strain OCB 456 that overexpressed the Pediococcus Idh gene (SEQ ID NO: 149, encoding SEQ ID NO: 50)
**Figure 3****:** Eda variant of RuMP cycle for the generation of lactate
**Figure 4****:** Fba variant of RuMP cycle for the generation of lactate

The present invention is now described in more detail below.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory).

### Bacterium of the invention

As indicated above, the present invention is based on the unexpected and surprising finding that methylotrophic bacteria can efficiently produce lactate through the expression of a polypeptide having lactate dehydrogenase activity.

The present invention thus provides in a first aspect a genetically engineered methylotrophic bacterium which has been modified to have an increased protein expression of a polypeptide having lactate dehydrogenase activity compared to an otherwise identical bacterium that does not carry said modification.

A "polypeptide having lactate dehydrogenase activity" is a polypeptide that catalyzes the reaction: lactate + NAD(+) <=> pyruvate + NADH. Non-limiting examples of such polypeptides are provides in SEQ ID Nos: 1 to 130. The polypeptide having lactate dehydrogenase activity may by a polypeptide having L-lactate dehydrogenase activity or a polypeptide having D-lactate dehydrogenase activity. A polypeptide having L-lactate dehydrogenase activity (EC 1.1.1.27) is a polypeptide that catalyzes the reaction: (S)-lactate + NAD(+) <=> pyruvate + NADH. Non-limiting examples of such polypeptides are provided in SEQ ID NO: 49 to 130. A polypeptide having D-lactate dehydrogenase activity (EC 1.1.1.28) is a polypeptide that catalyzes the reaction: (R)-lactate + NAD(+) <=> pyruvate + NADH. Non-limiting examples of such polypeptides are provided in SEQ ID NO: 1 to 48.

Polypeptides having lactate dehydrogenase activity are encoded in the genomes of a wide range of organisms. The polypeptide having lactate dehydrogenase may be derived from the same species as the bacterium in which it is expressed or may be derived from a species different to the one in which it is expressed (i.e. it is heterologous). According to some embodiments, the polypeptide having lactate dehydrogenase activity is derived from the same species as the bacterium in which it is expressed. According to some embodiments, the polypeptide having lactate dehydrogenase activity is derived from a species different from the one in which it is expressed (i.e. it is heterologous).

The polypeptide having lactate dehydrogenase activity may be a functional variant of a naturally occurring polypeptide having lactate dehydrogenase activity, i.e. it may be a polypeptide having lactate dehydrogenase activity which differs from the naturally occurring polypeptide having lactate dehydrogenase activity in the amino acid composition. Such functional variant may comprise an amino acid sequence which has at least 70%, such as at least 75%, at least 80%, at least 85%, at least 90% of at least 95%, sequence identity with the naturally occurring polypeptide and has lactate dehydrogenase activity.

According to some embodiments, the bacterium of the present invention expresses a heterologous polypeptide having lactate dehydrogenase activity.

By "increased protein expression" it is meant that the amount of the polypeptide having lactate dehydrogenase activity produced by the thus modified bacterium is increased compared to an otherwise identical bacterium that does not carry said modification. More particularly, by "increased expression" it is meant that the amount of the polypeptide having lactate dehydrogenase activity produced by the thus modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical bacterium that does not carry said modification. The amount of protein in a given cell can be determined by any suitable quantification technique known in the art, such as ELISA, Immunohistochemistry, or Western Blotting.

An increase in protein expression may be achieved by any suitable means well-known to those skilled in the art. For example, an increase in protein expression may be achieved by increasing the number of copies of a nucleotide sequence encoding the polypeptide having lactate dehydrogenase activity in the bacterium, such as by introducing into the bacterium at least one exogenous nucleic acid molecules comprising at least one nucleotide sequence encoding said polypeptide having lactate dehydrogenase activity.

Thus, according to some embodiments, the bacterium of the present invention comprises at least one exogenous nucleic acid molecule (such as a vector) comprising at least one nucleotide sequence encoding the polypeptide having lactate dehydrogenase activity.

Suitably, the exogenous nucleic acid molecule comprises at least one transcriptional unit comprising, from 5' to 3', a promoter that is functional in the bacterium to cause the production of an mRNA molecule and that is operably linked to a nucleotide sequence encoding said polypeptide having lactate dehydrogenase activity, and a transcriptional terminator sequence. The exogenous nucleic acid molecule may comprise at least two transcriptional units each comprising, from 5' to 3', a promoter that is functional in the bacterium to cause the production of an mRNA molecule and that is operably linked to a nucleotide sequence encoding said polypeptide, and a transcriptional terminator sequence. The transcriptional units may have the same type of promoter or different types of promoter.

The exogenous nucleic acid molecule may be a DNA construct, such as an expression cassette or a vector. The exogenous nucleic acid molecule may thus be a vector, such as an expression vector, or part of such vector, such as an expression cassette comprised by such vector. Normally, such a vector remains extrachromosomal within the bacterial cell which means that it is found outside of the genome of the bacterial cell. Alternatively, the exogenous nucleic acid molecule may be stably integrated into the genome of the bacterium (e.g., by random or targeted insertion). Particularly, the exogenous nucleic acid molecule may be an expression cassette stably integrated into the genome of the bacterium (e.g., by random or targeted insertion).

An increase in protein expression may also be achieved by the integration of at least a second copy of the endogenous gene encoding the polypeptide having lactate dehydrogenase activity into the genome of the bacterium.

An increase in protein expression may also be achieved by increasing the strength of the promoter operably linked to the endogenous gene encoding the polypeptide having lactate dehydrogenase activity, e.g. by replacing the native promoter with a promoter that enables higher expression and overproduction of polypeptide compared to the native promoter. The promoters that can be used include natural promoters from *Bacillus subtilis, Bacillus amyloliquefaciens* or similar, such as P43, P15, Pveg, Pylb, PgroES, PsigX, PtrnQ, Ppst, PsodA, PrpsF, PlepA, PliaG, PrpsF, Ppst, PfusA, PsodA, Phag as well as artificial promoters active in *Bacillus subtilis* or inducible *Bacillus subtilis* promoters, such as PmtIA, Pspac, PxylA, PsacB, or similar. Further examples include natural promoters from *Corynebacterium,* such as P CP_2454, Ptuf and Psod, natural promoters from *E. coli,* such as T7, ParaBAD, Plac, Ptac and Ptrc, and the promoter P F1 derived from the corynephage BFK20.

An increase in protein expression may also be achieved by modifying the ribosome binding site on the mRNA molecule encoding the polypeptide having lactate dehydrogenase activity. By modifying the sequence of the ribosome binding site, the translation initiation rate may be increased, thus increasing translation efficiency.

A polypeptide having lactate dehydrogenase activity for use according to the invention may for instance be a polypeptide having lactate dehydrogenase activity selected from the group consisting of: i) a polypeptide comprising an amino acid sequence of any one of SEQ ID NOs: 1 to 130; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 130.

According to some embodiments, the polypeptide having lactate dehydrogenase activity comprises amino acid sequence having at least 70%, such as at least 75%, sequence identity with the amino acid sequence of any one of SEQ ID NOs: 1 to 130. According to some embodiments, the polypeptide having lactate dehydrogenase activity comprises an amino acid sequence having at least 80%, such as at least 85%, sequence identity with the amino acid sequence of any one of SEQ ID NOs: 1 to 130. According to some embodiments, the polypeptide having lactate dehydrogenase activity comprises an amino acid sequence having at least 90%, such as at least 95%, sequence identity with the amino acid sequence of any one of SEQ ID NOs: 1 to 130. According to some embodiments, the polypeptide having lactate dehydrogenase activity comprises the amino acid sequence of any one of SEQ ID NOs: 1 to 130.

According to some embodiments, the "polypeptide having lactate dehydrogenase activity" is a polypeptide having L-lactate dehydrogenase activity.

A polypeptide having L-lactate dehydrogenase activity for use according to the invention may for instance be a polypeptide having lactate dehydrogenase activity selected from the group consisting of: i) a polypeptide comprising an amino acid sequence of any one of SEQ ID NOs: 49 to 130; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity with the amino acid sequence of any one of SEQ ID NOs: 49 to 130.

According to some embodiments, the polypeptide having lactate dehydrogenase activity comprises amino acid sequence having at least 70%, such as at least 75%, sequence identity with the amino acid sequence of any one of SEQ ID NOs: 49 to 130. According to some embodiments, the polypeptide having lactate dehydrogenase activity comprises an amino acid sequence having at least 80%, such as at least 85%, sequence identity with the amino acid sequence of any one of SEQ ID NOs: 49 to 130. According to some embodiments, the polypeptide having lactate dehydrogenase activity comprises an amino acid sequence having at least 90%, such as at least 95%, sequence identity with the amino acid sequence of any one of SEQ ID NOs: 49 to 130. According to some embodiments, the polypeptide having lactate dehydrogenase activity comprises the amino acid sequence of any one of SEQ ID NOs: 49 to 130.

According to some embodiments, the "polypeptide having lactate dehydrogenase activity" is a polypeptide having D-lactate dehydrogenase activity.

A polypeptide having D-lactate dehydrogenase activity for use according to the invention may for instance be a polypeptide having lactate dehydrogenase activity selected from the group consisting of: i) a polypeptide comprising an amino acid sequence of any one of SEQ ID NOs: 1 to 48; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 48.

According to some embodiments, the polypeptide having lactate dehydrogenase activity comprises amino acid sequence having at least 70%, such as at least 75%, sequence identity with the amino acid sequence of any one of SEQ ID NOs: 1 to 48. According to some embodiments, the polypeptide having lactate dehydrogenase activity comprises an amino acid sequence having at least 80%, such as at least 85%, sequence identity with the amino acid sequence of any one of SEQ ID NOs: 1 to 48. According to some embodiments, the polypeptide having lactate dehydrogenase activity comprises an amino acid sequence having at least 90%, such as at least 95%, sequence identity with the amino acid sequence of any one of SEQ ID NOs: 1 to 48. According to some embodiments, the polypeptide having lactate dehydrogenase activity comprises the amino acid sequence of any one of SEQ ID NOs: 1 to 48.

According to some embodiments, the polypeptide having lactate dehydrogenase activity comprises amino acid sequence having at least 70%, such as at least 75%, sequence identity with SEQ ID NO: 1. According to some embodiments, the polypeptide having lactate dehydrogenase activity comprises an amino acid sequence having at least 80%, such as at least 85%, sequence identity with SEQ ID NO: 1. According to some embodiments, the polypeptide having lactate dehydrogenase activity comprises an amino acid sequence having at least 90%, such as at least 95%, sequence identity with SEQ ID NO: 1. According to some embodiments, the polypeptide having lactate dehydrogenase activity comprises the amino acid sequence of SEQ ID NO: 1.

Techniques for determining lactate dehydrogenase activity are well known to the skilled person.. The lactate dehydrogenase activity may for instance be determined in accordance with the following method:
The cells expressing lactate dehydrogenase are centrifuged and lysed to release the intracellular contents using B-PER (Thermo Scientific) according to manufacturer instructions. Briefly, 4 mL B-PER is mixed with 1 g wet cell biomass and incubated for 15 minutes at room temperature. After the cells have been disrupted, the lysate is clarified using centrifugation. The clear cell lysate is used for determination of lactate dehydrogenase activity. The enzymatic reaction is performed at 37°C. The reaction contains 2.8 mL of 0.13 mM (β NADH) prepared in 100 mM Sodium phosphate buffer (pH 7.5) and 0.1 mL of 34 mM sodium pyruvate prepared in 100 mM Sodium phosphate buffer (pH 7.5). The components are mixed, and A340 is measured until constant. After stabilization, 0.1 mL cell free extract is added to the reaction, and mixed. The decrease in A340 is continuously measured for 5 minutes. The linear slope of the reaction is used to calculate the lactate dehydrogenase activity according to the formula U/ml =(ΔA340/min)^{∗}3^{∗}dilution factor/(6.22^{∗}0.1).

In order to overcome certain drawbacks in bioprocesses, such as to avoid mass cell lysis, the bacterium of the present invention may be further modified to decrease the expression and/or activity of an endogenous polypeptide having polyphosphate kinase activity in bacteria.

Thus, according to some embodiments, a bacterium of the present invention has been further modified to have a decreased expression and/or activity of an endogenous polypeptide having polyphosphate kinase activity compared to an otherwise identical bacterium that does not carry said modification.

A "polypeptide having polyphosphate kinase activity" is a polypeptide that catalyzes the reaction: ATP + (phosphate)ₙ <=> ADP + (phosphate)ₙ₊₁ (EC 2.7.4.1). Polyphosphate kinase (PPK), which is encoded by the *ppk* gene, is highly conserved in many bacteria, including methylotrophs such as *Methylobacillus flagellatus* and *Methylobacillus glycogenes,* and plays a crucial role in the ability of bacteria to adapt to nutritional stringencies and environmental stresses. Non-limiting examples of an endogenous polypeptide having polyphosphate kinase activity are provided in SEQ ID NOs: 156, 158, 160, 162 and 164.

According to some embodiments, the endogenous polypeptide having polyphosphate kinase activity comprises an amino acid sequence having at least 70%, such as at least 75%, sequence identity with the amino acid sequence set forth in any one of SEQ ID NOs: 156, 158, 160, 162 and 164. According to some embodiments, the endogenous polypeptide having polyphosphate kinase activity comprises an amino acid sequence having at least 80%, such as at least 85%, sequence identity with the amino acid sequence set forth in in any one of SEQ ID NOs: 156, 158, 160, 162 and 164. According to some embodiments, the endogenous polypeptide having polyphosphate kinase activity comprises an amino acid sequence having at least 90%, such as at least 95%, sequence identity with the amino acid sequence set forth in any one of SEQ ID NOs: 156, 158, 160, 162 and 164.

According to some embodiments, the endogenous gene encoding the polypeptide having polyphosphate kinase activity comprises a nucleic acid sequence having at least 70%, such as at least 75%, sequence identity with the nucleic acid sequence set forth in any one of SEQ ID NOs: 157, 159, 161, 163 and 165. According to some embodiments, the endogenous gene encoding the polypeptide having polyphosphate kinase activity comprises a nucleic acid sequence having at least 80%, such as at least 85%, sequence identity with the nucleic acid sequence set forth in any one of SEQ ID NOs: 157, 159, 161, 163 and 165. According to some embodiments, the endogenous gene encoding the polypeptide having polyphosphate kinase activity comprises a nucleic acid sequence having at least 90%, such as at least 95%, sequence identity with the nucleic acid sequence set forth in any one of SEQ ID NOs: 157, 159, 161, 163 and 165.

According to some embodiments, the endogenous polypeptide having polyphosphate kinase activity comprises an amino acid sequence having at least 70%, such as at least 75%, sequence identity with the amino acid sequence set forth in SEQ ID NO: 156. According to some embodiments, the endogenous polypeptide having polyphosphate kinase activity comprises an amino acid sequence having at least 80%, such as at least 85%, sequence identity with the amino acid sequence set forth in SEQ ID NO: 156. According to some embodiments, the endogenous polypeptide having polyphosphate kinase activity comprises an amino acid sequence having at least at least 90%, such as at least 95%, sequence identity with the amino acid sequence set forth in SEQ ID NO: 156.

According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 157. According to some embodiments, the endogenous gene encoding the polypeptide having polyphosphate kinase activity comprises a nucleic acid sequence having at least at least 80%, such as at least 85%, sequence identity with the nucleic acid sequence set forth in SEQ ID NO: 157. According to some embodiments, the endogenous gene encoding the polypeptide having polyphosphate kinase activity comprises a nucleic acid sequence having at least at least 90%, such as at least 95%, sequence identity with the nucleic acid sequence set forth in SEQ ID NO: 157.

According to some embodiments, the bacterium of the invention may be modified to have a decreased expression of an endogenous polypeptide having polyphosphate kinase activity compared to an otherwise identical bacterium that does not carry said modification.

According to some embodiments, the bacterium of the invention may be modified to have a decreased expression of an endogenous polypeptide having polyphosphate kinase activity compared to an otherwise identical bacterium that does not carry said modification. The expression level of the endogenous polypeptide having polyphosphate kinase activity may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the bacterium of the invention may be modified to have a decreased expression level of the endogenous gene encoding said endogenous polypeptide having polyphosphate kinase activity compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the endogenous gene encoding said polypeptide having polyphosphate kinase activity has been inactivated, such as by deletion of part of or the entire gene sequence.

According to some embodiments, the endogenous gene encoding said polypeptide having polyphosphate kinase activity has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said polypeptide having polyphosphate kinase activity is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said polypeptide having polyphosphate kinase activity is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said polypeptide.

According to some embodiments, the expression of said endogenous polypeptide having polyphosphate kinase activity is decreased by way of inhibition.

Inhibition of the expression of the said endogenous polypeptide may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said endogenous polypeptide having polyphosphate kinase activity is decreased (e.g., inhibited) by transcriptional and/or translational repression of the endogenous gene encoding said polypeptide.

According to some embodiments, the expression of endogenous polypeptide having polyphosphate kinase activity is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypetide. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific mRNA. Exemplary types of RNAi molecules include microRNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of an endogenous polypeptide having polyphosphate kinase activity compared to an otherwise identical bacterium that does not carry said modification.

A decrease of the activity of the polypeptide having polyphosphate kinase activity may be achieved by any suitable means known in the art. For example, the activity may be decreased by introducing one or more mutations in the active site of the polypeptide resulting in the reduction or loss of activity. Thus, according to some embodiments, the activity of the endogenous polypeptide having polyphosphate kinase activity is decreased by at least one active-site mutation resulting in the reduction or loss of activity. At least one active-site mutation may, for example, be at least one non-conservative amino acid substitution.

By way of example, if the activity of the endogenous the polypeptide having polyphosphate kinase activity is to be decreased in *Methylobacillus flagellatus*, the at least one active-site mutation may occur at any one of positions R379, S384, F492, P511, R568, R625, Q679, H439, and H458 in the amino acid sequence set forth in SEQ ID NO: 156 which form part of the active site. In case of orthologous polypeptides having polyphosphate kinase activity, the at least one active-site mutation may be at a position which corresponds to any one of positions R379, S384, F492, P511, R568, R625, Q678, H439, and H458 in the amino acid sequence set forth in SEQ ID NO: 156.

By way of another example, if the activity of the endogenous the polypeptide having polyphosphate kinase activity is to be decreased in *Methylobacillus glycogenes,* the at least one active-site mutation may occur at any one of positions R79, S84, F192, P211, R268, R325, Q378, H139, in the amino acid sequence set forth in SEQ ID NO: 158, which form part of the active site. In case of orthologous polypeptides having polyphosphate kinase activity, the at least one active-site mutation may be at a position which corresponds to any one of positions R79, S84, F192, P211, R268, R325, Q378, H139, H158 in the amino acid sequence set forth in SEQ ID NO: 158.

By way of another example, if the activity of the endogenous the polypeptide having polyphosphate kinase activity is to be decreased in *Methylobacillus rhizosphaerae,* the at least one active-site mutation may occur at any one of positions R379, S384, F492, P511, R568, R625, Q678, H439 and H458 in the amino acid sequence set forth in SEQ ID NO: 160, which form part of the active site. In case of orthologous polypeptides having polyphosphate kinase activity, the at least one active-site mutation may be at a position which corresponds to any one of positions R379, S384, F492, P511, R568, R625, Q678, H439 and H458 in the amino acid sequence set forth in SEQ ID NO: 160.

By way of another example, if the activity of the endogenous the polypeptide having polyphosphate kinase activity is to be decreased in *Methylobacterium organophilum,* the at least one active-site mutation may occur at any one of positions R392, S397, F505, P524, R581, R643, H452 and H471 in the amino acid sequence set forth in SEQ ID NO: 162, which form part of the active site. In case of orthologous polypeptides having polyphosphate kinase activity, the at least one active-site mutation may be at a position which corresponds to any one of positions R392, S397, F505, P524, R581, R643, H452 and H471 in the amino acid sequence set forth in SEQ ID NO: 162.

By way of another example, if the activity of the endogenous the polypeptide having polyphosphate kinase activity is to be decreased in *Methylorubrum extorquens,* the at least one active-site mutation may occur at any one of positions R451, S456, F564, P583, R640, R702, H511 and H530 in the amino acid sequence set forth in SEQ ID NO: 164, which form part of the active site. In case of orthologous polypeptides having polyphosphate kinase activity, the at least one active-site mutation may be at a position which corresponds to any one of positions R451, S456, F564, P583, R640, R702, H511 and H530 in the amino acid sequence set forth in SEQ ID NO: 164.

The resistance of such bacterium against cell lysis may be further improved, especially under certain conditions such as carbon limitation, by decreasing the expression and/or activity of an endogenous polypeptide having Acyl-homoserine-lactone (AHL) synthase activity.

Thus, according to some embodiments, the bacterium of the present invention may be further modified to have a decreased expression and/or activity of an endogenous polypeptide having Acyl-homoserine-lactone (AHL) synthase activity compared to an otherwise identical bacterium that does not carry said modification.

A "polypeptide having Acyl-homoserine-lactone (AHL) synthase activity" is a polypeptide that catalyzes the reaction: An acyl-[acyl-carrier-protein] + S-adenosyl-L-methionine <=> [acyl-carrier-protein] + S-methyl-5'-thioadenosine + an N-acyl-L-homoserine lactone (EC 2.3.1.184). Acyl-homoserine lactones (AHLs) are small signaling molecules used by many Gram-negative bacteria for coordinating their behavior as a function of their population density. This process, based on the biosynthesis and the sensing of such molecular signals, and referred to as Quorum Sensing (QS), regulates various gene expressions, including growth, virulence, biofilms formation, and toxin production. Non-limiting examples of an endogenous polypeptide having Acyl-homoserine-lactone (AHL) synthase activity are provided in SEQ ID NOs: 168, 170, 172, 174, 176, 178 and 180.

According to some embodiments, the endogenous polypeptide having Acyl-homoserine-lactone (AHL) synthase activity comprises an amino acid sequence having at least 70%, such as at least 75%, sequence identity with the amino acid sequence set forth in any one of SEQ ID NOs: 168, 170, 172, 174, 176, 178 and 180. According to some embodiments, the endogenous polypeptide having Acyl-homoserine-lactone (AHL) synthase activity comprises an amino acid sequence having at least 80%, such as at least 85%, sequence identity with the amino acid sequence set forth in any one of SEQ ID NOs: 168, 170, 172, 174, 176, 178 and 180. According to some embodiments, the endogenous polypeptide having Acyl-homoserine-lactone (AHL) synthase activity comprises an amino acid sequence having at least 90%, such as at least 95%, sequence identity with the amino acid sequence set forth in any one of SEQ ID NOs: 168, 170, 172, 174, 176, 178 and 180.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in any one of SEQ ID NOs: 169, 171, 173, 175, 177, 179 and 181. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in any one of SEQ ID NOs: 169, 171, 173, 175, 177, 179 and 181. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in any one of SEQ ID NOs: 169, 171, 173, 175, 177, 179 and 181.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 168. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 168. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 168.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 169. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 169. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 169.

According to some embodiments, the bacterium of the invention may be modified to have a decreased expression of an endogenous polypeptide having Acyl-homoserine-lactone (AHL) synthase activity compared to an otherwise identical bacterium that does not carry said modification.

According to some embodiments, the bacterium of the invention may be modified to have a decreased expression of an endogenous polypeptide having Acyl-homoserine-lactone (AHL) synthase activity compared to an otherwise identical bacterium that does not carry said modification. The expression level of the endogenous polypeptide having Acyl-homoserine-lactone

(AHL) synthase activity may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the bacterium of the invention may be modified to have a decreased expression level of the endogenous gene encoding said endogenous polypeptide having Acyl-homoserine-lactone (AHL) synthase activity compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the endogenous gene encoding said polypeptide having Acyl-homoserine-lactone (AHL) synthase activity has been inactivated, such as by deletion of part of or the entire gene sequence.

According to some embodiments, the endogenous gene encoding said polypeptide having Acyl-homoserine-lactone (AHL) synthase activity has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said polypeptide having Acyl-homoserine-lactone (AHL) synthase activity is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said polypeptide having Acyl-homoserine-lactone (AHL) synthase activity is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said polypeptide.

According to some embodiments, the expression of said endogenous polypeptide having Acyl-homoserine-lactone (AHL) synthase activity is decreased by way of inhibition.

Inhibition of the expression of the said endogenous polypeptide may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said endogenous polypeptide having Acyl-homoserine-lactone (AHL) synthase activity is decreased (e.g., inhibited) by transcriptional and/or translational repression of the endogenous gene encoding said polypeptide.

According to some embodiments, the expression of endogenous polypeptide having Acyl-homoserine-lactone (AHL) synthase activity is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypetide. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific mRNA. Exemplary types of RNAi molecules include microRNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of an endogenous polypeptide having Acyl-homoserine-lactone (AHL) synthase activity compared to an otherwise identical bacterium that does not carry said modification.

A decrease of the activity of the polypeptide having Acyl-homoserine-lactone (AHL) synthase activity may be achieved by any suitable means known in the art. For example, the activity may be decreased by introducing one or more mutations in the active site of the polypeptide resulting in the reduction or loss of activity. Thus, according to some embodiments, the activity of the endogenous polypeptide having Acyl-homoserine-lactone (AHL) synthase activity is decreased by at least one active-site mutation resulting in the reduction or loss of activity. At least one active-site mutation may, for example, be at least one non-conservative amino acid substitution.

As further detailed in Example 7, the present inventors have observed an unexpectedly high lactate titer when expressing a lactate dehydrogenase in a methylotrophic bacterium which utilizes the Eda-dependent RuMP pathway for methanol assimilation, such as *Methylobacillus flagellutes.* Accordingly, it will be advantageous to (over-)express a lactate dehydrogenase in a methylotrophic bacterium which expresses a RuMP cycle, and more specifically an Eda (2-keto-3-deoxy-phosphogluconate aldolase)-dependent RuMP cycle.

Thus, according to some embodiments, the bacterium of the present invention expresses a RuMP cycle, preferably an Eda (2-keto-3-deoxy-phosphogluconate aldolase)-dependent RuMP cycle.

A methylotrophic bacterium which expresses a RuMP cycle is a bacterium which expresses enzymes involved in the RuMP cycle. For example, a methylotrophic bacterium which expresses a RuMP cycle is a bacterium which expresses at least the following enzymes: a polypeptide having 3-hexulose-6-phosphate synthase activity (EC 4.1.2.43) and a polypeptide having 6-phospho-3-hexuloisomerase activity (EC 5.3.1.27). The methylotrophic bacterium may inherently (i.e. natively) express a RuMP cycle or may be modified to express a RuMP cycle by using, e.g., DNA recombination techniques.

As used herein, a "polypeptide having 3-hexulose-6-phosphate synthase activity" means a polypeptide that catalyzes the reaction: D-arabino-hex-3-ulose 6-phosphate <=> D-ribulose 5-phosphate + formaldehyde (EC 4.1.2.43). A non-limiting example of such polypeptide is provided in SEQ ID NO: 135 and variants thereof having at least 70%, such as at least 80%, at least 85%, at least 90%, at least 95% or at least 97%, sequence identity therewith.

As used herein, a "polypeptide having 6-phospho-3-hexuloisomerase activity" means a polypeptide that catalyzes the reaction: D-arabino-hex-3-ulose 6-phosphate <=> D-fructose 6-phosphate (EC 5.3.1.27). A non-limiting example of such polypeptide is provided in SEQ ID NO: 136 and variants thereof having at least 70%, such as at least 80%, at least 85%, at least 90%, at least 95% or at least 97%, sequence identity therewith.

According to some embodiments, the bacterium of the present invention expresses a polypeptide having 3-hexulose-6-phosphate synthase activity and a polypeptide having 6-phospho-3-hexuloisomerase activity.

According to some embodiments, the bacterium of the present invention expresses an Eda (2-keto-3-deoxy-phosphogluconate aldolase)-dependent RuMP cycle.

A methylotrophic bacterium which expresses an Eda (2-keto-3-deoxy-phosphogluconate aldolase)-dependent RuMP cycle is a bacterium which expresses, besides enzymes involved in the RuMP cycle, notably a polypeptide having 3-hexulose-6-phosphate synthase activity and a polypeptide having 6-phospho-3-hexuloisomerase activity, also a polypeptide having phosphoglucoisomerase activity (EC 5.3.1.9) and a polypeptide having 2-keto-3-deoxy-phosphogluconate aldolase activity (EC 4.1.2.14). The methylotrophic bacterium may inherently (i.e. natively) express a polypeptide having phosphoglucoisomerase activity and a polypeptide having 2-keto-3-deoxy-phosphogluconate aldolase activity or may be modified to express said polypeptides by using, e.g., DNA recombination techniques.

As used herein, a "polypeptide having phosphoglucoisomerase activity" means a polypeptide that catalyzes the reaction: Alpha-D-glucose 6-phosphate <=> beta-D-fructofuranose 6-phosphate (EC 5.3.1.9). A non-limiting example of such polypeptide is provided in SEQ ID NO: 131 and variants thereof having at least 70%, such as at least 80%, at least 85%, at least 90%, at least 95% or at least 97%, sequence identity therewith.

As used herein, a "polypeptide having 2-keto-3-deoxy-phosphogluconate aldolase activity" means a polypeptide that catalyzes the reaction: 2-dehydro-3-deoxy-6-phosphate-D-gluconate <=> pyruvate + D-glyceraldehyde 3-phosphate (EC 4.1.2.14). Non-limiting examples of such polypeptide are provided in SEQ ID NOs: 132 to 134 and variants thereof having at least 70%, such as at least 80%, at least 85%, at least 90%, at least 95% or at least 97%, sequence identity therewith.

According to some embodiments, the bacterium of the present invention (further) expresses a polypeptide having phosphoglucoisomerase activity and a polypeptide having 2-keto-3-deoxy-phosphogluconate aldolase activity.

According to some embodiments, the bacterium of the present invention expresses a polypeptide having 3-hexulose-6-phosphate synthase activity, a polypeptide having 6-phospho-3-hexuloisomerase activity, a polypeptide having phosphoglucoisomerase activity and a polypeptide having 2-keto-3-deoxy-phosphogluconate aldolase activity.

Non-limiting examples of methylotrophic bacteria which inherently (i.e. natively) express a RuMP cycle, and more specifically an Eda (2-keto-3-deoxy-phosphogluconate aldolase)-dependent RuMP cycle include *Methylobacilli* such as *Methylobacillus flagellatus* and *Methyllobacillus glycogenes.*

Generally, a bacterium as referred to herein may be any suitable methylotrophic bacterium. The bacterium may be Gram-positive or Gram-negative. Preferably, the bacterium is a Gram-negative bacterium.

According to some embodiments, the bacterium of the present invention is a mesophilic, methylotrophic bacterium.

According to some embodiments, the bacterium of the present invention belongs to the family *Methylophilaceae* or *Methylobacteriaceae.*

According to some embodiments, the bacterium of the present invention belongs to the family *Methylophilaceae.*

According to some embodiments, the bacterium of the present invention belongs to the family *Methylobacteriaceae.*

According to some embodiments, the bacterium of the present invention belongs to the genus *Methylobacillus, Methylobacterium or Methylorubrum.*

According to some embodiments, the bacterium of the present invention belongs to the genus *Methylobacillus* or *Methylobacterium.*

According to some embodiments, the bacterium of the present invention belongs to the genus *Methylobacterium or Methylorubrum.*

According to some embodiments, the bacterium of the present invention is selected from *Methylobacillus flagellatus*, *Methylobacillus glycogenes, Methylobacillus pratensis, Methylobacillus rhizosphaerae, Methylobacillus gramineus, Methylobacillus arboreus, Methylobacillus caricics, Methylobacillus methilovorans, Methylobacillus sp, Methylobacterium extorquens, Methylobacterium organophilum and Methylorubrum extorquens.*

According to some embodiments, the bacterium of the present invention belongs to the genus *Methylobacillus.*

According to some embodiments, the bacterium of the present invention is selected from *Methylobacillus flagellatus*, *Methylobacillus glycogenes, Methylobacillus pratensis, Methylobacillus rhizosphaerae, Methylobacillus gramineus, Methylobacillus arboreus, Methylobacillus caricics, Methylobacillus methilovorans,* and *Methylobacillus sp.*

According to some embodiments, the bacterium of the present invention is *Methylobacillus flagellutes.*

According to some embodiments, the bacterium of the present invention is *Methylobacillus glycogenes.*

According to some embodiments, the bacterium of the present invention is *Methylobacillus rhizosphaerae.*

### Method of the invention

The present invention also provides methods for producing lactate comprising cultivating a bacterium according to the invention under suitable culture conditions. The method may further comprise collecting lactate from the culture medium.

According to some embodiments, the present invention provides a method for producing L-lactate.

According to some embodiments, the present invention provides a method for producing D-lactate.

According to some embodiments, the present invention provides a method for producing L-lactate and D-lactate.

The culture medium employed may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The carbon source may be any suitable carbon substrate known in the art, and in particularly any carbon substrate commonly used in the cultivation of methylotrophic bacteria and/or fermentation. A carbon source of particular interest is a reduced one-carbon compound, such as methanol, methane, formate, or methylamine, or a multi-carbon compound that contains no carbon-carbon bonds, such as dimethyl ether and dimethylamine. Thus, according to some embodiments, the culture medium comprises methanol as a carbon source. The concentration of methanol in the culture medium may generally be between in the range from about 0.5% w/v to about 4 % w/v, such as from about 2% w/v to about 4 % w/v. According to some embodiments, the concentration of methanol in the culture medium is in the range from about 2.5% w/v to about 3.5% w/v.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used.

Suitably, the bacterium is cultivated under suitable conditions for the production of the desired product. Suitable conditions for culturing the respective bacterium are well known to the skilled person. Typically, a bacterium is cultured at a temperature ranging from about 20 to about 45°C, such as from about 30 to about 38°C, such as at about 37°C. The cultivation can be preferably performed under aerobic conditions, such as by a shaking culture, by a stirring culture or in a bioreactor with aeration, at a temperature of about 20 to about 45 °C, such as about 30 to 38 °C, preferably at about 37°C. The pH of the culture is usually above 5, such as in a range from about 6 to about 8, preferably from about 6.5 to about 7.5, more preferably from about 6.8 to about 7.2. The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as cells can be removed from the culture medium by centrifugation or membrane filtration. The biochemical compound can be collected by conventional method for isolation and purification chemical compounds from a medium. Well-known purification procedures include, but are not limited to, centrifugation or filtration, precipitation, ion exchange, chromatographic methods such as e.g. ion exchange chromatography or gel filtration chromatography, and crystallization methods. The method may further comprise collecting lactate from the culture medium.

The present invention also provides a biochemical compound obtainable by a method as detailed above.

### Certain other definitions

The term "mesophilic" as used herein in the context of a bacterium means that the bacterium grows best in moderate temperature with an optimum growth range from 20 to 45 °C.

The term "methylotrophic" as used herein in the context of a bacterium means that the bacterium can use reduced one-carbon compounds, such as methanol, methane, formate, or methylamine, as the carbon source for their growth, and multi-carbon compounds that contain no carbon-carbon bonds, such as dimethyl ether and dimethylamine.

"Polypeptide" and "protein" are used interchangeably herein to denote a polymer of at least two amino acids covalently linked by an amide bond, regardless of length or post-translational modification (e.g., glycosylation, phosphorylation, lipidation, myristoylation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a host cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genes that are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it is present or which is found in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression" of a gene it is meant that the amount of the transcription product, respectively the amount of the polypeptide (e.g., enzyme) encoded by said gene produced by the modified bacterium is decreased compared to an otherwise identical bacterium that does not carry said modification. More particularly, by "decreased expression" of a gene it is meant that the amount of the transcription product, respectively the amount of the polypeptide (e.g., enzyme) encoded by said gene produced by the modified bacterium is decreased by at least 10%, such as at least 20%, at least 30%, at least 40%, at least 50% at least 60%, at least 70%, at least 80%, at least 90% or at least 100%, compared to an otherwise identical bacterium that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

Expression of a gene can be decreased by introducing a mutation into the gene in the genome of the bacterium so that the intracellular activity of the polypeptide encoded by the gene is decreased as compared to an otherwise identical bacterium that does not carry said mutation. Mutations which result in a decreased expression of the gene include the replacement of one nucleotide or more to cause an amino acid substitution in the polypeptide encoded by the gene (missense mutation), introduction of a stop codon (nonsense mutation), deletion or insertion of nucleotides to cause a frame shift, insertion of a drug-resistance gene, or deletion of a part of the gene or the entire gene (Qiu and Goodman, 1997; Kwon et al., 2000). Expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The lambda-red mediated gene replacement is a particularly suitable method to inactive one or more genes as described herein.

"Inactivating", "inactivation" and "inactivated", when used in the context of a gene or gene cluster, means that the gene or gene cluster in question no longer expresses a functional protein. It is possible that the modified DNA region is unable to naturally express the gene or gene cluster due to the deletion of a part of or the entire sequence of the gene or gene cluster, the shifting of the reading frame of the gene or gene cluster, the introduction of missense/nonsense mutation(s), or the modification of the regulatory region of the gene or gene cluster, including sequences controlling gene expression, such as a promoter, enhancer, attenuator, ribosome- binding site, etc. Preferably, a gene or gene cluster of interest is inactivated by deletion of a part of or the entire sequence of the gene or gene cluster, such as by gene replacement. Inactivation may also be accomplished by introducing or expressing a rare-cutting endonuclease able to selectively inactivating by DNA cleavage, preferably by double-strand break, the gene or gene cluster of interest. A "rare-cutting endonuclease" within the context of the present invention includes transcription activator-like effector (TALE) nucleases, meganucleases, zing-finger nucleases (ZFN), and RNA-guided endonucleases.

The presence or absence of a gene or gene cluster in the genome of a bacterium can be detected by well-known methods, including PCR, Southern blotting, and the like. In addition, the level of gene expression can be estimated by measuring the amount of mRNA transcribed from the gene or gene cluster using various well-known methods, including Northern blotting, quantitative RT-PCR, and the like. The amount of the polypeptide encoded by the gene or gene cluster can be measured by well-known methods, including SDS-PAGE followed by an immunoblotting assay (Western blotting analysis), and the like.

As used herein, "decreased", "decreasing" or "decrease of" expression of a polypeptide (such as a polypeptide as described herein) means that the expression of said polypeptide in a modified bacterium is reduced compared to the expression of said polypeptide in an otherwise identical bacterium that does not carry said modification (control). The expression of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). More particularly, "decreased", "decreasing" or "decrease of" expression of a polypeptide means that the amount of the polypeptide in the modified bacterium is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical bacterium that does not carry said modification (control). The expression or amount of a polypeptide in a bacterium can be determined by any suitable means know in the art, including techniques such as ELISA, Immunohistochemistry, Western Blotting or Flow Cytometry.

As used herein, "abolished" expression of a polypeptide (such as a polypeptide as described herein) means that the expression of said polypeptide in a modified bacterium is not detectable compared to the expression of said polypeptide in an otherwise identical bacterium that does not carry said modification (control).

As used herein, "decreased", "decreasing" or "decrease of" activity of a polypeptide (such as an enzyme as described herein) means that the catalytic activity of said polypeptide in a modified bacterium is reduced compared to the catalytic activity of said polypeptide in an otherwise identical bacterium that does not carry said modification (control). The activity of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). The activity of a polypeptide in a bacterium can be determined by any suitable protein and enzyme activity assay.

"Expression" includes any step involved in the production of a polypeptide (e.g., encoded enzyme) including, but not limited to, transcription, post-transcriptional modification, translation, post-translational modification, and secretion.

As used herein, "regulatory region" of a gene or gene cluster refers to a nucleic acid sequence that affect the expression of a coding sequence. Regulatory regions are known in the art and include, but are not limited to, promoters, enhancers, transcription terminators, polyadenylation sites, matrix attachment regions and/or other elements that regulate expression of a coding sequence.

"Substitution" or "substituted" refers to modification of the polypeptide by replacing one amino acid residue with another, for instance the replacement of an Serine residue with a Glycine or Alanine residue in a polypeptide sequence is an amino acid substitution. When used with reference to a polynucleotide, "substitution" or "substituted" refers to modification of the polynucleotide by replacing one nucleotide with another, for instance the replacement of a cytosine with a thymine in a polynucleotide sequence is a nucleotide substitution.

"Conservative substitution", when used with reference to a polypeptide, refers to a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

As used herein, "vector" refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "% sequence identity" and "percent identity" refers to sequence identity between a nucleotide sequence and a reference nucleotide sequence or between an amino acid sequence and a reference amino acid sequence. Sequence identity can be determined by comparing a position in each sequence which may be aligned for purposes of comparison. When a position in the compared sequence is occupied by the same base or amino acid, then the molecules are identical at that position. A degree of identity between nucleotide or amino acid sequences is a function of the number of identical or matching nucleotides or amino acids at positions shared by the nucleotide or amino acid sequences, respectively. Various alignment algorithms and/or programs may be used to calculate the identity between two sequences, including FASTA or BLAST which are available as a part of the GCG sequence analysis package (University of Wisconsin, Madison, Wis.), and can be used with default settings.

"Reference sequence" or "reference amino acid sequence" refers to a defined sequence to which another sequence is compared. In the context of the present invention a reference amino acid sequence may, for example, be an amino acid sequence set forth in SEQ ID NO: 1.

As used herein, the term "about" means plus or minus 10% of the numerical value of the number with which it is being used.

Where a numerical limit or range is stated herein, the endpoints are included. Also, all values and sub ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components or groups thereof.

Having generally described this invention, a further understanding can be obtained by reference to certain specific examples, which are provided herein for purposes of illustration only, and are not intended to be limiting unless otherwise specified.

### Examples

### Example 1: Bioinformatic analysis

While investigating the potential of *Methylobacillus flagellatus* for use in industrial biotechnology, we noticed it produced significantly higher titers of lactic acid under certain bioreactor fermentation conditions compared to other common overflow products such as acetate. This was unexpected because the published *Methylobacillus flagellatus* genome (Chistoserdova L. et al., 2007) contained no annotation for a lactate dehydrogenase enzyme. To identify which gene was responsible for lactic acid production the amino acid sequences of two well-characterized lactate dehydrogenase genes from *Escherichia coli,* IdhA (SEQ ID NO: 9) and IldD (SEQ ID NO: 49), were used to perform a BLAST search against the *Methylobacillus flagellatus* genome. The search resulted in only three hits, summarized in Table 1. The hit described as "2-hydroxyacid dehydrogenase" with the locus tag Mfla_0399 (SEQ ID NO: 137, encoding SEQ ID NO: 1) was deemed as the only plausible candidate.

**Table 1: Summary of BLAST search of E. coli lactate dehydrogenases against the Methylobacillus flagellatus genome.**

| **Query** | **Hit description** | **Query Coverage** | **Identity** |
|---|---|---|---|
| **IdhA** | 2-hydroxyacid dehydrogenase | 100% | 51% |
| **IdhA** | phosphoglycerate dehydrogenase | 70% | 30% |
| **IldD** | FMN-binding glutamate synthase family protein | 28% | 34% |

The amino acid sequence of this gene was used to perform a BLAST search against all known genomes in the *Methylobacillus* genus, resulting in a similar "2-hydroxyacid dehydrogenase" hit in each genome (SEQ ID NOs: 2-8) with a high degree of similarity. A protein alignment using the Clustal Omega service was performed on the identified sequenced, revealing a high degree of conservation across all strains, with several 100% conserved regions and an overall similarity of over 84%.

### Example 2: Expression of novel identified enzyme and production of lactate in methylotrophs

To test whether the identified gene (Mfla_0399, SEQ ID NO: 137) is responsible for lactate production by *Methylobacillus flagellatus*, we prepared constructs that enable controlled gene expression in *M. flagellatus* and other *Methylobacilli.* The gene was amplified from the genome of *M. flagellatus* by PCR and cloned into an expression vector under the control of the IPTG-inducible lacO/trc promotor, using the NEB Hi-Fi cloning kit according to manufacturer instructions. The prepared plasmids were checked by sequencing and transformed into *M. flagellatus* via electroporation to create strain OCB 354.

To test lactate production, the transformants were cultivated in 250 mL shake flasks in a mineral medium mineral medium containing methanol, KH₂PO₄, Na₂HPO₄, MgSO₄, NH₄SO₄, and trace elements. The shake flasks were inoculated with 5 mL liquid overnight culture. After 4 h of growth, 0.5 g/L IPTG was added to induce gene expression. The cultures were sampled after 24 h of growth. Lactate was measured by HPLC.

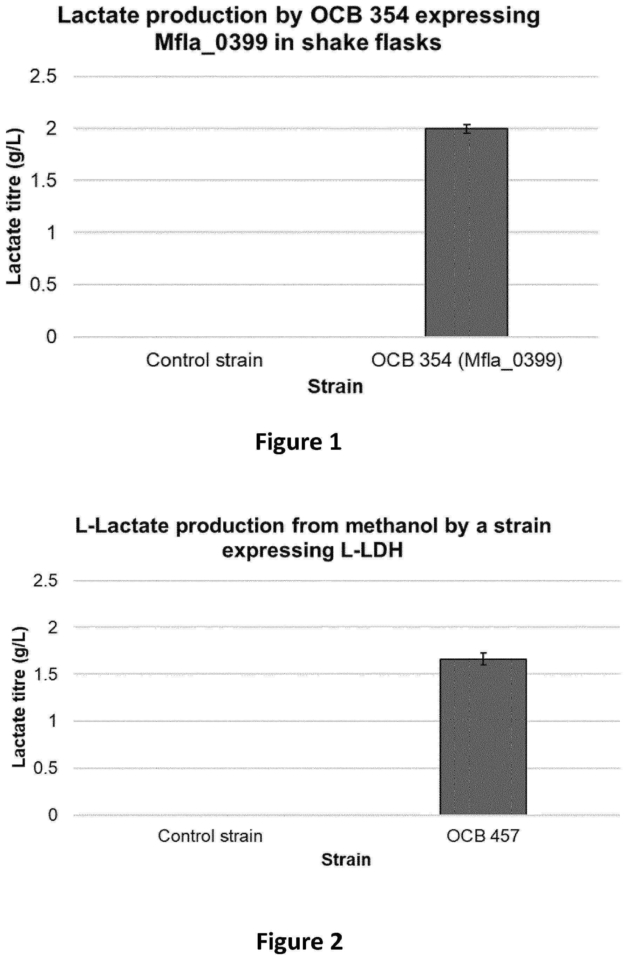

The strain OCB 354, expressing the Mfla_0399 gene (SEQ ID NO: 137) produced 2 g/L lactate from methanol after 24 h of cultivation **(****Figure 1****).** Under the same conditions, the control strain that did not express Mfla_0399 did not produce detectable amounts of lactic acid. This confirmed that Mfla_0399 is responsible for converting methanol into lactate in *M. flagellatus.*

### Example 3: Expression of L-lactate dehydrogenase and production of L-lactate from methanol

To enable L-lactate synthesis from methanol, we expressed a L-lactate dehydrogenase and expressed it in *M. flagellatus.* The construct and strains were prepared as the described in Example 2. The gene sequence was taken *Pediococcus* (strain OCB 457, SEQ ID NO: 50) and harmonized for gene expression in *M. flagellatus.*

To test lactate production, the transformants were cultivated in 250 mL shake flasks in a mineral medium mineral medium containing methanol, KH₂PO₄, Na₂HPO₄, MgSO₄, NH₄SO₄, and trace elements. The shake flasks were inoculated with 5 mL liquid overnight culture. After 4 h of growth, 0.5 g/L IPTG was added to induce gene expression. The cultures were sampled after 24 h of growth. Lactate was measured by HPLC.

The strain OCB 457 produced approximately 1.6 g/L lactate from methanol after 24 h of cultivation **(****Figure 2****).** Under the same conditions, the control strain that did not express any lactate dehydrogenase gene did not produce detectable amounts of lactic acid.

### Example 4: Determination of lactic acid chirality.

We tested the chirality of the lactate produced by strains OCB 354 and OCB 457. We tested the clarified cell broth at the end of the fermentation by using the enzymatic L-lactic acid and D-lactic acid specific kits from Megazyme (K-LATE and K-DATE, respectively). The assays were performed according to the manufacturer instructions. We confirmed that the chirality of the lactate was determined by the enzyme. One racemate (either D-lactate or L-lactate) constituted more than 97% of the lactate, depending on the LDH enzyme (Table 2).

**Table 2: Chirality determination of the lactic acid produced by various strains**

| **Strain** | **Enzyme** | **D-lactate** | **L-lacate** |
|---|---|---|---|
| **OCB 354** | Mfla_0399 D-LDH | 98.3% | 1.7% |
| **OCB 457** | Pediococcus L-LDH | 3.0% | 97.0% |

### Example 5: Identification and selection of other lactate dehydrogenase enzymes

Lactate dehydrogenases are very common and well-characterized enzymes present in a broad variety of organisms, from archaea to humans. While the conversion of pyruvate always requires reducing power, it can be provided in the form of different reducing equivalent cofactors. NADH dependent lactate dehydrogenases are the most biotechnologically relevant. To identify examples of lactate dehydrogenases covering a broad phylogenetic range, the E.C. numbers 1.1.1.27 (L-lactate dehydrogenases) and 1.1.1.28 (D-lactate dehydrogenases) were used to perform a search in the UniProt public protein database, generating 16 836 and 3 719 hits respectively. The complete results were downloaded and processed to group them by phylum of the source organism. The sequences were filtered for outliers regarding their length and a single example from each phylum was extracted, for a total of 41 D-lactate specific (SEQ ID NOs: 12-48, protein only) and 84 L-lactate specific (SEQ ID NOs: 51-130, protein only) NADH lactate dehydrogenase sequences.

### Example 7: Metabolic probing

When we expressed the lactate dehydrogenase, we observed an unexpectedly high lactate titer in the strain OCB 354. We therefore probed the central carbon metabolism of the organism by deleting several genes of the glycolytic and RuMP pathway.

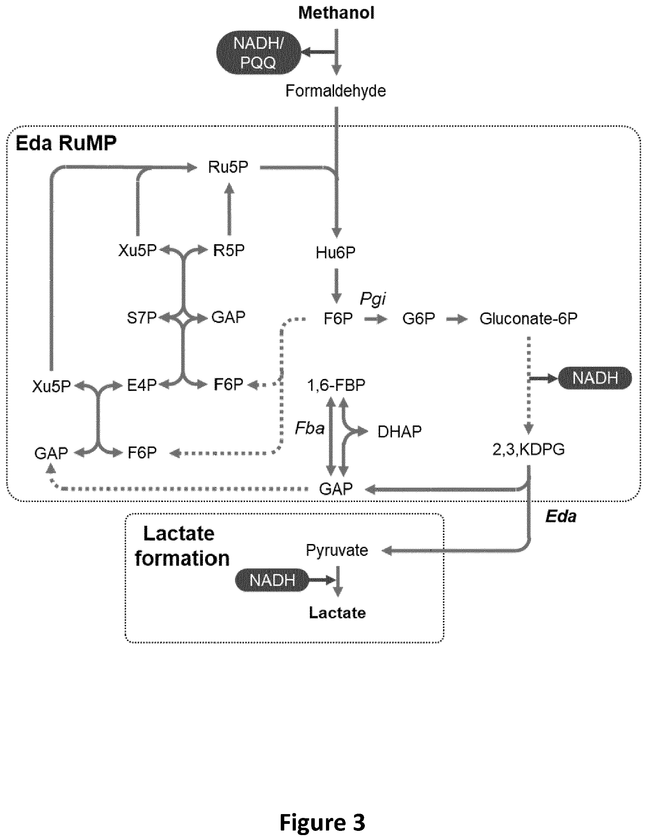

While we were successful in deleting a variety of the genes, we could not delete the phosphoglucoisomerase (pgi) gene (Mfla_1325, gene sequence SEQ ID NO: 150, protein sequence SEQ ID NO: 131) in the strain. We investigated the genome of the organism for more details and found that this is critical for the Eda (2-keto-3-deoxy-phosphogluconate aldolase)-dependent RuMP cycle used by M. flagellates to extract C3 compounds from the RuMp cycle. This enzyme (Mfla_0760, protein sequence SEQ ID NO: 132, gene sequence SEQ ID NO: 151) splits the C6 compound 2-keto-3-deoxy-phosphogluconate (2,3-KDPG) into glyceraldehyde-3P (GAP) and pyruvate. GAP is used to replenish the RuMP cycle for the next cycle of methanol assimilation, while pyruvate exits the cycle **(****Figure 3****).** Coincidentally, pyruvate is the metabolite needed for lactate formation by lactate dehydrogenase. This means that the strain utilizing the Eda-RuMP pathway for methanol assimilation has a surprisingly high, and direct metabolic flux towards pyruvate, which results in high lactate production observed in the strain OCB 354.

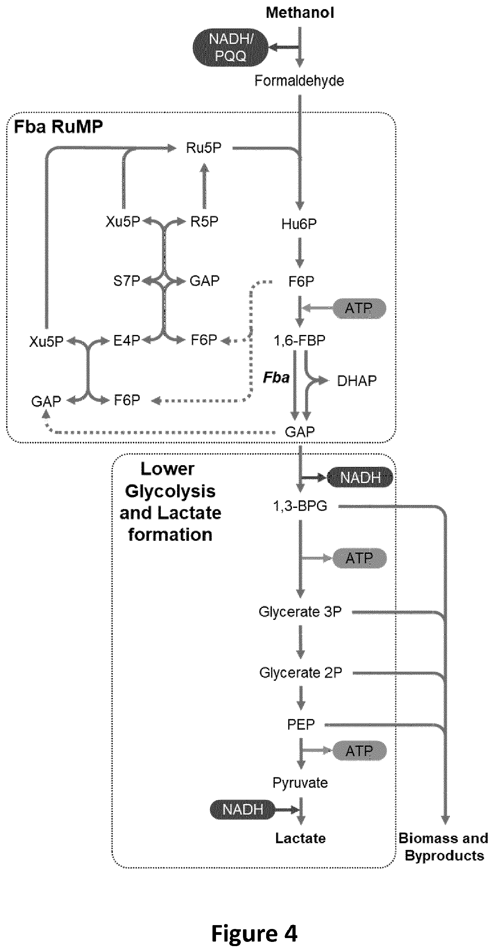

The alternative RuMP cycle depends on the fructose bisphospate aldolase (Fba) to cleave the C6 intermediates of the RuMP cycle. This generates two GAP moieties, one of which is used for RuMP regeneration, and the other exists the cycle **(****Figure 4****).** The GAP is then converted to pyruvate via a series or metabolic reactions. This could potentially be used for lactate production. However, most of the pathway intermediates can be used for biomass and byproduct formation, making the Fba-RuMP cycle less efficient for lactate formation from methanol, and the Eda-RuMP superior in comparison.

The combination of the Eda-dependent RuMP cycle and over-expression of lactate dehydrogenase is therefore beneficial for efficient lactate formation in strain OCB 354. A similar effect of increased lactate production is also expected when the lactate dehydrogenase (over-)expression is combined with the expression of heterologous Eda enzymes, for example from *Pseudomonas putida* or *E. coli* (SEQ ID NOs: 133 and 134, respectively).

### List of references cited in the description

Chistoserdova, L. et al. (2007) 'Genome of Methylobacillus flagellatus, Molecular Basis for Obligate Methylotrophy, and Polyphyletic Origin of Methylotrophy', Journal of Bacteriology. American Society for Microbiology Journals, 189(11): 4020-4027.

Qiu Z and Goodman MF: The Escherichia coli polB locus is identical to dinA, the structural gene for DNA polymerase II. Characterization of Pol II purified from a polB mutant. J Biol Chem. 1997, 272(13): 8611-8617.

Kwon DH, Peña JA, Osato MS, Fox JG, Graham DY, Versalovic J: Frameshift mutations in rdxA and metronidazole resistance in North American Helicobacter pylori isolates. J Antimicrob Chemother 2000, 46(5): 793-796

Datsenko KA, Wanner BL: One-step inactivation of chromosomal genes in Escherichia coli K-12 using PCR products. Proc Natl Acad Sci U S A 2000, 97:6640-6645

## Claims

1. A genetically engineered methylotrophic bacterium which has been modified to have an increased protein expression of a polypeptide having lactate dehydrogenase activity compared to an otherwise identical bacterium that does not carry said modification.

2. The bacterium according to claim 1, wherein the bacterium comprises at least one exogenous nucleic acid molecule comprising at least one nucleotide sequence encoding the polypeptide having lactate dehydrogenase activity.

3. The bacterium according to claim 1 or 2, wherein the polypeptide having lactate dehydrogenase activity is selected from the group consisting of: i) a polypeptide comprising an amino acid sequence of any one of SEQ ID NOs: 1 to 130 and ii) a polypeptide comprising an amino acid sequence, which has at least 70% sequence identity with the amino acid sequence of any one of SEQ ID NOs: 1 to 130.

4. The bacterium according to any one of claims 1 to 3, wherein the polypeptide having lactate dehydrogenase activity is selected from the group consisting of: i) a polypeptide comprising the amino acid sequence of SEQ ID NO: 1; and ii) a polypeptide comprising an amino acid sequence, which has at least 70% sequence identity with the amino acid sequence of SEQ ID NO: 1.

5. The bacterium according to any one of claims 1 to 4, which has been further modified to have a decreased expression and/or activity of an endogenous polypeptide having polyphosphate kinase activity compared to an otherwise identical bacterium that does not carry said modification.

6. The bacterium according to any one of claims 1 to 5, which has been modified to have a decreased expression of an endogenous polypeptide having polyphosphate kinase activity compared to an otherwise identical bacterium that does not carry said modification.

7. The bacterium according to any one of claims 1 to 6, which has been further modified to have a decreased expression and/or activity of an endogenous polypeptide having Acyl-homoserine-lactone (AHL) synthase activity compared to an otherwise identical bacterium that does not carry said modification.

8. The bacterium according to any one of claims 1 to 7, which has been modified to have a decreased expression of an endogenous polypeptide having Acyl-homoserine-lactone (AHL) synthase activity compared to an otherwise identical bacterium that does not carry said modification.

9. The bacterium according to any one of claims 1 to 8, which expresses a RuMP cycle.

10. The bacterium according to any one of claims 1 to 9, which expresses an Eda (2-keto-3-deoxy-phosphogluconate aldolase)-dependent RuMP cycle.

11. The bacterium according to any one of claims 1 to 10, which expresses a polypeptide having phosphoglucoisomerase activity and a polypeptide having 2-keto-3-deoxy-phosphogluconate aldolase activity.

12. The bacterium according to any one of claims 1 to 11, wherein said bacterium belongs to the genus *Methylobacillus, Methylobacterium or Methylorubrum.*

13. The bacterium according to any one of claims 1 to 12, wherein said bacterium is of the genus *Methylobacillus.*

14. The bacterium according to any one of claims 1 to 13, wherein said bacterium is *Methylobacillus flagellatus* or *Methylobacillus glycogenes.*

15. Method for producing lactate comprising cultivating a bacterium according to any one of claims 1 to 14 under suitable culture conditions.
